# EUROPÄISCHE PATENTSCHRIFT

(11) **EP 1 886 701 B1**
(45) Veröffentlichungstag und Bekanntmachung des Hinweises auf die Patenterteilung: **23.06.2010**
(21) Anmeldenummer: 07013432.5
(22) Anmeldetag: 10.07.2007
(51) Int. Cl.: A61L 31/18, A61L 31/08, A61K 49/04

(54) **Markerkomposit für medizinische Implantate**
Marker composite for medical implants
Marqueurs composites pour implants médicaux

(30) Priorität: 07.08.2006 DE 102006038233
(43) Veröffentlichungstag der Anmeldung: 13.02.2008
(73) Patentinhaber: Biotronik VI Patent AG, 6341 Baar (CH)
(72) Erfinder: Borck, Alexander, Dr., 91086 Aurachtal (DE); Korzuschnik, Ellen, 91077 Hetzles (DE); Harder, Claus, Dr., 91080 Uttenreuth (DE); Müller, Heinz, Dr., 91052 Erlangen (DE)
(74) Vertreter: Lindner-Vogt, Karin L.

(56) Entgegenhaltungen:
- WO-A-03/039612
- DE-A1- 10 361 942

## Beschreibung

Die Erfindung betrifft ein Markerkomposit für medizinische Implantate aus einem biokorrodierbaren metallischen Werkstoff sowie ein medizinisches Implantat, das mit einem solchen Markerkomposit beschichtet oder befüllt ist.

### Stand der Technik

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

Zur radiologischen intra- und postoperativen Positionsüberwachung werden Implantate, sofern sie nicht bereits aus einem ausreichend radioopaken Material bestehen, mit einem Marker versehen. Die Röntgensichtbarkeit des Markers hängt von seinen Abmessungen und Röntgenabsorptionskoeffizienten ab. Letzterer ist wiederum abhängig vom Energiebereich der Röntgenstrahlung: im medizinischen Bereich liegt dieser in der Regel bei 60 bis 120 keV, bei koronarer Anwendung wird typischerweise im Bereich von 80 bis 100 keV gearbeitet. Mit steigender Ordnungszahl im Periodensystem und steigender Dichte des Materials wird der Röntgenabsorptionskoeffizienten in der Regel größer. Die Gegenwart des Markers sollte nicht die Funktionalität des Implantats einschränken und/oder Ausgangspunkt für Entzündungs- oder Abstoßungsreaktionen des Körpers sein. Als Markerwerkstoffe werden herkömmlich beispielsweise Edelmetalle, wie Gold und Platin, eingesetzt.

Die Marker liegen (i) als Vollmaterial, z. B. in Form einer Beschichtung, eines Bandes, eines Inlays oder eines mit dem Implantat fest verbundenen Formkörpers oder (ii) als in eine Trägermatrix eingebettete Pulver, in Form einer Beschichtung oder als Füllmaterial für eine Kavität im Implantat, vor. Variante (ii) kann herstellungstechnisch besonders einfach umgesetzt werden: Es wird ein gieß- oder sprühfähiges Gemisch aus der radioopaken Markerkomponente und dem als Trägermatrix agierenden Material, gegebenenfalls unter Zusatz eines Lösungsmittels, verarbeitet.

Implantate werden nach Erfüllung ihres therapeutischen Zweckes wieder operativ entfernt, sofern dies noch möglich ist, denn ein dauerhafter Verbleib der Implantate im Körper kann zu Entzündungs- oder Abstoßungsreaktionen führen. Eine Alternative zur Operation liegt in der Verwendung biokorrodierbarer Werkstoffen für das Implantat. Die Zahl biokorrodierbarer Werkstoffe auf der Basis von Polymeren oder Metallen ist stetig gewachsen. So sind unter anderem biokorrodierbare Metalllegierungen der Elemente Magnesium, Eisen und Wolfram bekannt. Beispielsweise beschreibt EP 1 270 023 eine Magnesiumlegierung, die sich für endovaskuläre und orthopädische Implantate eignet.

Die aus dem Stand der Technik bekannten biokorrodierbaren Metalllegierungen und Polymere für medizinische Implantate besitzen im medizintechnischen Energiebereich von 80 - 100 keV nur eine geringe Röntgensichtbarkeit. Zur postoperativen Überwachung des Heilungsfortschrittes oder zur Kontrolle minimal-invasiver Eingriffe ist aber gerade die Röntgendiagnostik ein wichtiges Instrumentarium. So werden z. B. seit einigen Jahren Stents in den Koronararterien bei der akuten Myokardinfarkttherapie platziert. Der Stent wird im Bereich der Läsion der koronaren Gefäßwand positioniert und soll eine Obstruktion der Gefäßwand nach deren Aufweitung verhindern. Der Vorgang der Positionierung und Expansion der Stents muss während der Prozedur durch den Kardiologen laufend überwacht werden.

Bei Implantaten aus biokorrodierbaren metallischen Werkstoffen auf der Basis von Magnesium, Eisen oder Wolfram bestehen erhöhte Anforderungen an das Markermaterial:
- der Marker sollte durch die korrosiven Prozesse nicht frühzeitig vom Grundkörper des Implantates getrennt werden, um einer Fragmentbildung und damit der Gefahr der Embolisation vorzubeugen;
- der Marker sollte schon bei geringen Materialdicken eine hinreichende Röntgendichte besitzen und
- das Markermaterial sollte möglichst keinen oder allenfalls einen geringen Einfluss auf die Degradation des Grundkörpers haben.

DE 103 61 942 A1 beschreibt einen radioopaken Marker für medizinische Implantate, der 10 bis 90 Gewichtsprozente einer biokorrodierbaren Basiskomponente, insbesondere aus der Gruppe der Elemente Magnesium, Eisen und Zink, enthält. Weiterhin enthält der Marker 10 bis 90 Gew.% eines oder mehrerer radioopaker Elemente aus der Gruppe 1, Au, Ta, Y, Nb, Mo, Ru, Rh, Ba, La, Ce, Pr, Nd, Sm, Eu, Gd, Tb, Dy, Ho, Er, Tm, Yb, Lu, Hf, Ta, W, Re, Os und Bi als Markerkomponente. Die beschriebenen Marker eignen sich demnach prinzipiell für den Einsatz in biokorrodierbaren Implantaten, insbesondere solchen aus biokorrodierbaren Magnesiumlegierungen.

Bei Einsatz von Markern aus metallischen Werkstoffen auf biokorrodierbaren metallischen Grundkörpern besteht jedoch das besondere Problem, dass sich aufgrund von elektrochemischen Wechselwirkungen zwischen den beiden metallischen Materialien die Degradation des Grundkörpers in einem Kontaktbereich zwischen Marker und Grundkörper ändert, d. h. in der Regel beschleunigt ist.

### Zusammenfassung der Erfindung

Der vorliegenden Erfindung liegt die Aufgabe zugrunde, ein Markerkomposit für medizinische Implantate aus einem biokorrodierbaren metallischen Werkstoff bereitzustellen, das die zuvor genannten Kriterien der Materialwahl erfüllt. Insbesondere sollte sich das Markerkomposit einfach verarbeiten lassen, eine hohe Biokompatibilität und ein gutes Haftvermögen zum Grundkörper des Implantats aufweisen sowie zumindest in Teilen biokorrodierbar sein.

Nach einem ersten Aspekt der Erfindung wird diese Aufgabe durch das erfindungsgemä-ße Markerkomposit für medizinische Implantate aus einem biokorrodierbaren metallischen Werkstoff gelöst. Das Markerkomposit zeichnet sich dadurch aus, dass es eine Zusammensetzung wie folgt aufweist:
(i) 1 - 40, bevorzugt 10 - 40, Gewichtsteile einer Trägermatrix mit einem Schmelzpunkt größer oder gleich 43 °C, die zu 90 Gew.% oder mehr aus Triglyceriden besteht; und
(ii) 60 - 99, bevorzugt 60 - 90, Gewichtsteile einer radioopaken Markerkomponente, die in die Trägermatrix eingebettet ist.

Es hat sich überraschender Weise gezeigt, dass der Einsatz einer Triglyceridmatrix für die Aufnahme radioopaker Markerkomponenten besonders vorteilhaft ist: Das Material ist naturidentisch oder natürlichen Ursprungs und daher hochgradig biokompatibel, es ist biologisch abbaubar, besitzt ein gutes Haftvermögen auf metallischen Oberflächen, ist kostengünstig beziehbar und verarbeitungstechnisch leicht zu handhaben.

Der Begriff Triglyceride (auch Triacylglyceride, Triacylglycerole) ist eine Sammelbezeichnung für Ester des Glycerols (Glyceride) in denen alle drei Hydroxy-Gruppen mit Fettsäuren verestert sind. Die erfindungsgemäß eingesetzten Triglyceride sind natürlichen Ursprungs oder sogenannte strukturierte Triglyceride. Unter strukturierten Triglyceriden versteht man Triglyceride, deren natürliche Verteilung der Fettsäure-Reste an den primären Positionen des Glycerol-Gerüstes (sn-1, sn-2, sn-3) durch chemische, biochemische, pflanzenzüchterische oder gentechnische Maßnahmen gezielt verändert wurde, um diesen Fetten bestimmte lebensmitteltechnologische, biochemische oder physiologische Eigenschaften zu verleihen. Triglyceride sind hydrophob und polymorph, d.h. sie kristallisieren in verschiedenen Modifikationen, die als γ, α, β' oder. β bezeichnet werden. Am stabilsten ist die β-Form. Der Schmelzpunkt der Triglyceride hängt von der Fettsäure-Zusammensetzung sowie von der Position im Glycerid-Molekül ab. Die Anwesenheit cisungesättigter Fettsäuren erniedrigt in der Regel die Schmelztemperatur.

Die erfindungsgemäßen Triglyceride zeichnen sich nun dadurch aus, dass sie einen Schmelzpunkt der β-Form von 43 °C oder mehr besitzen. Hierdurch ist sichergestellt, dass die Trägermatrix auch bei hohem Fieber des Patienten fest bleibt und so der Gefahr einer durch Verlust des Markers induzierten Embolisation entgegengewirkt werden kann. Vorzugsweise liegt der Schmelzpunkt der β-Form im Temperaturintervall von 43 °C bis 100 °C. Triglyceride mit einem Schmelzpunkt der β-Form oberhalb von 100 °C lassen sich verarbeitungstechnisch nur schwer handhaben und besitzen ein im Vergleich zu niederschmelzenden Triglyceriden geringeres Haftvermögen.

Nach einer bevorzugten Ausführungsform besteht die Trägermatrix zu 90 Gew.% oder mehr aus einem hydrierten Sojaöl mit einem Anteil von Palmitinsäure an den Fettsäuren des Triglycerids im Bereich von 9 bis 16 Gew.% und einem Anteil von Stearinsäure an den Fettsäuren des Triglycerids im Bereich von 79 bis 89 Gew.%. Es hat sich gezeigt, dass eine Trägermatrix der genannten Zusammensetzung eine besonders einfache Verarbeitung zulässt und ein hohes Haftvermögen besitzt. Zudem weist das Material eine außergewöhnlich hohe Biokompatibilität auf. Vorzugsweise enthält die Trägermatrix 0,1 bis 20 Gew.%, bevorzugt 0,5 bis 20 Gew.%, Tocopherol als einen die Viskosität der Trägermatrix erniedrigenden Zusatz. Hierdurch wird die Verarbeitung vereinfacht.

Als radioopake Markerkomponenten können alle herkömmlichen, im Zusammenhang mit Implantaten genannten Materialien, wie Metalle oder anorganische Salze, Einsatz finden. Die Markerkomponente liegt in der Trägermatrix in gelöster oder suspendierter Form vor. Vorzugsweise ist die Markerkomponente ein Metallpulver, insbesondere mit einer mittleren Teilchengröße größer oder gleich 3 µm. Das Metallpulver ist vorzugsweise ein Element ausgewählt aus der Gruppe Gold, Iridium, Platin und Tantal. Der Einsatz feinpulvriger Markerkomponenten vereinfacht die Verarbeitung und Auftragung des Markerkomposits auf das Implantat beziehungsweise Einbringung des Markerkomposits in eine Kavität des Implantats. Die Trägermatrix verringert eine Kontaktfläche zwischen den metallischen Markerkomponenten und dem Grundkörper des Implantats, so dass unerwünschte Wechselwirkungen in Bezug auf das Korrosionsverhalten vermieden oder zumindest gemindert sind. Vorzugsweise wird der Bereich des Implantats, der den Marker tragen soll, mit einer kleinen Menge der Triglycerid-Trägermatrix vor Aufbringung des Markers beschichtet.

Vorzugsweise ist der biokorrodierbare metallische Werkstoff eine biokorrodierbare Legierung, ausgewählt aus der Gruppe Magnesium, Eisen und Wolfram; insbesondere ist der biokorrodierbare metallische Werkstoff eine Magnesiumlegierung. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Legierungen der Elemente Magnesium, Eisen oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut, ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-OberflächenVerhältnis, Konzentrationsunterschied sowie Strömungsgeschwindigkeit beeinflusst.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird.

Der Röntgenmarker liegt als Pulver, vorzugsweise mit einer mittleren Teilchengröße kleiner oder gleich 3 µm vor, wobei das Pulver in das als organische Trägermatrix agierende Triglycerid eingebettet ist. Der Vorteil liegt unter anderem in der Vereinfachung der Verarbeitung: Es kann eine Dispersion aus den beiden Komponenten des Markerkomposits - gegebenenfalls unter Zusatz eines geeigneten Lösungsmittels - hergestellt werden, die sich über herkömmliche Beschichtungsverfahren auf das Implantat auftragen lässt oder als Füllmaterial für eine Kavität im Implantat dient. Nach dem Abbau der biokorrodierbaren Trägermatrix verbleibt die pulverförmige Markerkomponente und wird vermutlich aufgrund seiner geringen Teilchengröße in extrazellulären Vesikeln gespeichert. Es ist davon auszugehen, dass eine derartige Einlagerung des Materials die Gefahr von Entzündungs- oder Abstoßungsreaktionen mindert.

Ein zweiter Aspekt der Erfindung liegt in der Bereitstellung eines medizinischen Implantats mit einem Röntgenmarker entsprechend den vorgenannten Ausführungen. Insbesondere ist dieses medizinische Implantat ein Stent, vorzugsweise ein Stent aus einer biokorrodierbaren Magnesiumlegierung.

### Ausführungsbeispiel

Ein Stent aus der biokorrodierbaren Magnesiumlegierung WE43 (93 Gew.% Magnesium, 4 Gew.% Yttrium [W] und 3 Gew.% Seltenerdmetalle [E]) wurde wie folgt mit einem Röntgenmarker beschichtet:
Es wurde eine Suspension von 500 mg hydrierten Sojaöl (erhältlich bei der Fa. Gustav Heess unter der Handelbezeichnung Hydriertes Sojaöl Ph. Eur. 5.0, IP; Fettsäuregewichtsanteile: Palmitinsäure 9 - 16 Gew.%, Stearinsäure 79 - 89 Gew.%, Ölsäure und Isomere max. 4 Gew.%, restliche Fettsäuren jeweils max. 1 Gew.%) und 47,5 g TaC-Pulver mit einer mittleren Teilchengröße von etwa 0.8 - 2 µm (erhältlich bei OSRAM SYLVANIA Products Inc.) unter Rühren und Erwärmen auf eine Temperatur von ca. 60°C angesetzt. Die warme Suspension wurde in eine Kavität im Stent dispergiert und anschließend auf Raumtemperatur abgekühlt.

## Patentansprüche

1. Markerkomposit für medizinische Implantate aus einem biokorrodierbaren metallischen Werkstoff, wobei das Markerkomposit die Zusammensetzung aufweist:
(i) 1 - 40 Gewichtsteile einer Trägermatrix mit einem Schmelzpunkt größer oder gleich 43 °C, die zu 90 Gew.% oder mehr aus Triglyceriden besteht; und
(ii) 60 - 99 Gewichtsteile einer radioopaken Markerkomponente, die in die Trägermatrix eingebettet ist.

2. Markerkomposit nach Anspruch 1, **dadurch gekennzeichnet, dass** das der biokorrodierbare metallische Werkstoff eine Magnesiumlegierung ist.

3. Markerkomposit nach einem der vorhergehenden Ansprüche, **dadurch gekennzeichnet, dass** das Implantat ein Stent ist.

4. Markerkomposit nach einem der vorhergehenden Ansprüche, **dadurch gekennzeichnet, dass** die Trägermatrix zu 90 Gew.% oder mehr aus einem hydrierten Sojaöl mit einem Anteil von Palmitinsäure an den Fettsäuren des Triglycerids im Bereich von 9 - 16 Gew.% und einem Anteil von Stearinsäure an den Fettsäuren des Triglycerids im Bereich von 79 - 89 Gew.% besteht.

5. Markerkomposit nach einem der vorhergehenden Ansprüche, **dadurch gekennzeichnet, dass** die Markerkomponente ein Metallpulver ist.

6. Markerkomposit nach Anspruch 5, **dadurch gekennzeichnet, dass** das Metallpulver eine mittlere Teilchengröße kleiner oder gleich 3 µm aufweist.

7. Markerkomposit nach Anspruch 5 oder 6, **dadurch gekennzeichnet, dass** das Metallpulver ein Element ausgewählt aus der Gruppe Au, Ir, Pt und Ta ist.

8. Markerkomposit nach einem der vorhergehenden Ansprüche, **dadurch gekennzeichnet, dass** die Trägermatrix 0,1-20 Gew.% Tocopherol als einen die Viskosität erniedrigenden Zusatz enthält.

9. Medizinisches Implantat, beschichtet und/oder befüllt mit einem Markerkomposit nach einem der vorhergehenden Ansprüche.

## Claims

1. A marker composite for medical implants of a biocorrodible metallic material, where the marker composite has the composition:
(i) 1-40 parts by weight of a carrier matrix with a melting point greater than or equal to 43°C, consisting of 90 wt% or more triglycerides; and
(ii) 60-99 parts by weight of a radiopaque marker component, which is embedded in the carrier matrix.

2. The marker composite according to claim 1, **characterized in that** the biocorrodible metallic material is a magnesium alloy.

3. The marker composite according to any one of the preceding claims, **characterized in that** the implant is a stent.

4. The marker composite according to any one of the preceding claims, **characterized in that** the carrier matrix consists of 90 wt% or more of a hydrogenated soy oil with palmitic acid being present in the range of 9-16 wt% of the fatty acids of the triglyceride and stearic acid being present in the range of 79-89 wt% of the fatty acids of the triglyceride.

5. The marker composite according to any one of the preceding claims, **characterized in that** the marker component is a metal powder.

6. The marker composite according to claim 5, **characterized in that** the metal powder has an average particle size less than or equal to 3 µm.

7. The marker composite according to claim 5 or 6, **characterized in that** the metal powder is an element selected from the group of Au, Ir, Pt and Ta.

8. The marker composite according to any one of the preceding claims, **characterized in that** the carrier matrix contains 0.1-20 wt% tocopherol as an additive for reducing viscosity.

9. A medical implant, coated and/or filled with a marker composite according to any one of the preceding claims.

## Revendications

1. Composite marqueur pour implants médicaux en matériau métallique biocorrodable, ce composite marqueur présentant la composition suivante:
(i) 1 à 40 parties en poids de matrice porteuse ayant un point de fusion supérieur ou égal à 43° C, qui est composée à 90 % en poids ou plus de triglycérides ; et
(ii) 60 à 99 parties en poids de composant marqueur radio-opaque qui est noyé dans la matrice porteuse.

2. Composite marqueur selon la revendication 1, **caractérisé en ce que** le matériau métallique biocorrodable est un alliage de magnésium.

3. Composite marqueur selon une des revendications précédentes, **caractérisé en ce que** l'implant est un stent.

4. Composite marqueur selon une des revendications précédentes, **caractérisé en ce que** la matrice porteuse est composée à 90 % en poids ou plus d'huile de soja hydrogénée ayant une teneur en acide palmitique au niveau des acides gras du triglycéride dans la fourchette de 9 à 16 % en poids et une teneur en acide stéarique au niveau des acides gras du triglycéride dans la fourchette de 79 à 89 % en poids.

5. Composite marqueur selon une des revendications précédentes, **caractérisé en ce que** le composant marqueur est une poudre métallique.

6. Composite marqueur selon la revendication 5, **caractérisé en ce que** la poudre métallique présente une taille moyenne de particules inférieure ou égale à 3 µm.

7. Composite marqueur selon la revendication 5 ou 6, **caractérisé en ce que** la poudre métallique est un élément sélectionné dans le groupe Au, Ir, Pt et Ta.

8. Composite marqueur selon une des revendications précédentes, **caractérisé en ce que** la matrice porteuse contient 0,1 à 20 % en poids de tocophérol servant d'additif réducteur de viscosité.

9. Implant médical, revêtu et/ou rempli d'un composite marqueur selon une des revendications précédentes.
